# EUROPEAN PATENT SPECIFICATION

(11) **EP 2 733 490 B1**
(45) Date of publication and mention of the grant of the patent: **22.02.2017**
(21) Application number: 12782401.9
(22) Date of filing: 10.05.2012
(51) Int. Cl.: G01N 33/574, G01N 33/90, C12Q 1/26

(54) **BIFUNCTIONAL TUMOUR DIAGNOSIS REAGENT AND METHOD THEREFOR**
BIFUNKTIONELLES TUMORDIAGNOSE-REAGENZ UND VERFAHREN DAFÜR
RÉACTIF BI-FONCTIONNEL ET PROCÉDÉ DE DIAGNOSTIC DE TUMEUR

(30) Priority: 12.05.2011 CN 201110122433
(43) Date of publication of application: 21.05.2014
(73) Proprietor: Institute of Biophysics Chinese Academy of Sciences, Chaoyang District, Beijing 100101 (CN)
(72) Inventor: YAN, Xiyun, Beijing 100101 (CN); LIANG, Minmin, Beijing 100101 (CN); FAN, Kelong, Beijing 100101 (CN); YANG, Dongling, Beijing 100101 (CN); LU, Di, Beijing 100101 (CN); FENG, Jing, Beijing 100101 (CN); DUAN, Demin, Beijing 100101 (CN)
(74) Representative: Zacco Denmark A/S
(86) International application number: PCT/CN2012/075291
(87) International publication number: WO 2012/152222

(56) References cited:
- MASAKI UCHIDA ET AL: "Targeting of Cancer Cells with Ferrimagnetic Ferritin Cage Nanoparticles", JOURNAL OF THE AMERICAN CHEMICAL SOCIETY, vol. 128, no. 51, 1 December 2006 (2006-12-01), pages 16626-16633, XP55150188, ISSN: 0002-7863, DOI: 10.1021/ja0655690
- LIZENG GAO ET AL: "Intrinsic peroxidase-like activity of ferromagnetic nanoparticles", NATURE NANOTECHNOLOGY, vol. 2, no. 9, 26 August 2007 (2007-08-26) , pages 577-583, XP55150181, ISSN: 1748-3387, DOI: 10.1038/nnano.2007.260
- JIA C P ET AL: "Nano-ELISA for highly sensitive protein detection", BIOSENSORS AND BIOELECTRONICS, ELSEVIER BV, NL, vol. 24, no. 9, 15 May 2009 (2009-05-15), pages 2836-2841, XP026053074, ISSN: 0956-5663, DOI: 10.1016/J.BIOS.2009.02.024 [retrieved on 2009-03-06]
- ZHIWEN TANG ET AL: "Enzyme-Mimic Activity of Ferric Nano-Core Residing in Ferritin and Its Biosensing Applications", ANALYTICAL CHEMISTRY, vol. 83, no. 22, 12 September 2011 (2011-09-12), pages 8611-8616, XP055150241, ISSN: 0003-2700, DOI: 10.1021/ac202049q
- FAN J ET AL: "Direct evidence for catalase and peroxidase activities of ferritin-platinum nanoparticles", BIOMATERIALS, ELSEVIER SCIENCE PUBLISHERS BV., BARKING, GB, vol. 32, no. 6, 1 February 2011 (2011-02-01), pages 1611-1618, XP027568279, ISSN: 0142-9612 [retrieved on 2010-12-21]

### SEQUENCE LISTING

<110> Institute Of Biophysics, Chinese Academy Of Sciences
   Institute of Geology and Geophysics, Chinese Academy of Sciences
<120> Bifunctional Tumor Diagnostic Reagent and Method
<130> FP130154US
<160> 3
<170> PatentIn version 3.3
<210> 1
   <211> 552
   <212> DNA
   <213> Homo sapiens
<400> 1
   atgacgaccg cgtccacctc gcaggtgcgc cagaactacc accaggactc agaggccgcc     60
   atcaaccgcc agatcaacct ggagctctac gcctcctacg tttacctgtc catgtcttac    120
   tactttgacc gcgatgatgt ggccttgaag aactttgcca aatactttct tcaccaatct    180
   catgaggaga gggaacatgc tgagaaactg atgaagctgc agaaccaacg aggtggccga    240
   atcttccttc aggatatcaa gaaaccagac tgtgatgact gggagagcgg gctgaatgcg    300
   atggagtgtg cattacattt ggaaaaaaat gtgaatcagt cactactgga actgcacaaa    360
   ctggccactg acaaaaatga cccccatttg tgtgacttca ttgagacaca ttacctgaat    420
   gagcaggtga aagccatcaa agaattgggt gaccacgtga ccaacttgcg caagatggga    480
   gcgcccgaat ccggcttggc ggaatatctc tttgacaagc acaccctggg agacagtgat    540
   aatgaaagct ag                                                        552
<210> 2
   <211> 25
   <212> DNA
   <213> artifical
<400> 2
   agtcgcccat atgacgaccg cgtcc 25
<210> 3
   <211> 26
   <212> DNA
   <213> artifical
<400> 3
   gccggatcct tagctttcat tatcac 26

## Description

### Technical Field

The present invention falls into an interdisciplinary field covering nano-technology, bionics, immunology and biomedicine. In particular, the present invention provides a tumor diagnostic reagent which integrates two functions, i.e., tumor specific identification and visualization. The present invention also provides a method for the diagnosis of cancer tissues and cancer cells using the said tumor diagnostic reagent.

### Background

Malignant tumor has become one of the common life-threatening diseases. Pathological section identification is one of the most precise and reliable method, which is recognized as the "Golden Standard" for tumor diagnosis in home and abroad (Shi, et al., (2008) Am. J. Clin. Pathol., 129:358-366; Taylor et al. (2006) Biotech Histochem., 81:3-12.; Larsson, et al., (1988) Immunocytochemistry: Theory and Practice. Boca Raton, FL: CRC Press 41-73). At present, the main staining method for a pathological section includes haematoxylin-eosin (HE) staining, immunohistochemistry and immunofluorescence. In HE staining, the chromatin in the nuclei is stained royal blue with haematoxylin, and the cytoplasm and the components in the extracellular matrix are stained red with eosin, so that the information about cell morphology is provided. As a result, based on the change of the cell morphology, a pathological physician can make a rough identification for the carcinogenesis of tissues and cells. Such a staining method is convenient and quick in manipulation, but it can only provide the change of cell morphology. With such relatively less information, it is not possible to make a precise judgment for more complicated and polytype tumors. In the immunohistochemistry or immunofluorescence method, a primary antibody binds to an antigen in the tissue to be detected, and then a fluorescent signal molecule-labeled or an enzyme-labeled secondary antibody or tertiary antibody binds to the primary antibody; subsequently, information about the location and semi-quantification of the antigen to be detected is provided by a fluorescent signal or color development through enzymatic substrate reaction, and pathological changes such as carcinogenesis, necrosis of the tissues and cells, and the infiltration of inflammatory cells and the like are further identified. The immunohistochemistry or immunofluorescence can provide detailed information about the distribution and amount of a specific antigen and the cell morphology, and is very helpful for thorough research of the pathology. However, the immune-staining requires several steps of incubation with the primary antibody, the secondary antibody, or even the tertiary antibody, repeated washings with PBS, labeling with an enzyme or a fluorescent molecule, which involves complex steps and a long processing time. Therefore, in order to improve the clinically pathological diagnosis efficiency and win time for the treatment of patients, it is necessary to investigate a reagent and a simple and quick method which is capable of providing abundant information for detecting cancerous tissues and cancerous cells.

Natural human ferritin is a spherical protein for iron storage, which is assembled from 24 heavy chain or light chain subunits at any ratio. The heavy chain subunit and the light chain subunit are highly homologous, and have a molecular weight of 21 kDa and 19 kDa, respectively (Theil, (1987) Annu. Rev. Biochem., 56:289-315). Ferritin has different subunit components in different human organs and tissues, with heavy chain subunits predominant in heart, and light chain subunits predominant in liver. The two types of subunits vary with the environmental requirements, which provides flexible supply of iron ions to the organism. However, only the heavy chain subunit is capable of converting Fe²⁺ to Fe³⁺ using oxygen so as to facilitate iron ions to successfully enter ferritin. Therefore, increasing the heavy chain subunit components can improve the cell's availability of iron, and increasing the light chain subunit components can improve the efficiency of iron storage. In the spherical shell of ferritin, iron ions form a crystalline with phosphates and hydroxyl ions, which has similar chemical properties to iron oxide hydrate in the minerals (Harrison, et al., (1996) Biochim. Biophys. Acta 1275:161-203; Levi, et al. (1988) J. Biol. Chem. 263:18086-18092; Ford, et al. (1984) Philos Trans. R. Soc. Lond B Biol. Sci. 304:551-565).

In addition to storing iron in cells, heavy chain subunit of ferritin is capable of specifically binding to activated lymphocytes, leukemic cells, cervical cancer cells, lymphoma cells, and the like (Fargion, et al. (1988) Blood 71:753-757; Moss, et al.(1992) J. Lab Clin. Med. 119:273; Bretscher, et al. (1983) EMBO J. 2:599-603; Takami, et al. (1986) Biochim. Biophys Acta 884:31-38; Fargion, et al. (1991) Blood, 78:1056-1061). Li et al. reported that transferrin receptor 1 (TfR1) on the cell surface, as a co-receptor of the heavy chain subunit of ferritin, mediates the interaction between ferritin and cells (Li, et al., (2010) Proc. Natl. Acad. Sci. U S A 107: 3505-3510). The transferrin receptor is a critical molecule for the cell to obtain iron element, and is essential for cell proliferation and rapid growth of tumor cells. Many tumors such as hepatocellular carcinoma, breast cancer, pancreatic cancer, stomach cancer, colon cancer, and the like have a high expression level of TfR1, so that TfR1 is a specific target for identification and treatment of tumors (Larrick, et al. (1979) J. Supramol. Struct.11:579-586; Daniels, et al. (2006) Clin. Immunol.,121:144-158; Ryschich, et al. (2004) Eur. J. Caner 40:1418-1422.). Since TfR1 is a co-receptor of the heavy chain subunit of ferritin, the heavy chain subunit of ferritin can be used to specifically identify tumors.

The natural protein shells having various sizes of nano-cavity structures mainly comprise apoferritin, heat shock protein (HSP, with a diameter of 12 nm), DNA-binding protein (DPs, with a diameter of 9 nm), pteridine synthase (LS, with a diameter of 15 nm) and viral protein shells such as cowpea chlorotic mottle virus (CCMV, with a diameter of 28 nm) and cowpea mosaic virus protein (CMV, with a diameter of 31 nm). Such a nano-cavity provides a good spatial structure for growing nanoparticles and efficiently preventing the aggregation of nanoparticles during synthesis. In view of such a specific structure, inorganic particles of various sizes can be synthesized in the cavity, and a controlled synthesis of nano-materials in different sizes, shapes and crystalline structures can be achieved (Turyanska L., et al. (2009) Small, 5:1738-1741; Meldrum FC., et al. (2009) Science, 257:522-523;Galvez N., et al. (2006) J. Mater. Chem., 16:2757-2761; Hennequin B., et al. (2008) Adv. Mater., 20:3592-3593; Yamashita I., et al. (2004) Chem. Lett., 33:1158-1159). Moreover, these protein shells having different structures can be easily chemically coupled or genetically fused to target molecules and signal molecules so as to enable a targeted identification and detection of cancer. For example, apoferritin enables a targeted identification of cancer cells by genetically fused to RGD (Uchida M., et al. (2006) J. Am. Chem. Soc., 128: 16626-16633), and enables *in vivo* tumor imagining by coupled to chemical label molecule Cy5.5 or nano-cavity loaded isotope ⁶⁴Cu and gadolinium (Lin X., et al. (2011) Nano Lett., 11: 814-819; Geninatti CS., et al. (2006) Cancer Res., 66: 9196-201.).

In 2007, our team found that iron oxide nanoparticles have a similar catalytic activity to peroxidases for the first time, i.e., in the presence of hydrogen peroxide, iron oxide nanoparticles can react with the substrates of horseradish peroxidase such as DAB and TMB to generate the same reaction products as peroxidase does. So, iron oxide nanoparticles have a similar catalytic activity to peroxidases. Based on the finding, we proposed the concept of iron oxide nanoparticle enzyme mimic (Gao L, et al., (2007) Nature Nanotech., 2:577-583; Chinese Patent Application No. 200610057413.9).

Further relevant art can be seen in Uchida M., et al. (2006), Targeting of Cancer Cells with Ferrimagnetic ferritin cage nanoparticles, J. Am. Chem. Soc., 128: 16626-16633, relates to protein cage architecture such as virus capsids and ferritins as versatile nanoscale platforms amenable to both genetic and chemical modification.

Gao L., et al. (2007), Intrinsic peroxidase-like activity of ferromagnetic nanoparticles, Nature Nanotech., 2:577-583, relates to nanoparticles containing magnetic materials, such as Fe₃O₄ that are particularly useful for imaging and separation techniques.

Jia, C. P., et al., (2009), Nano-ELISA for high sensitivity protein detection. Biosensors and Bioelectronics, 24(9), pp. 2836-2841, relates to a high sensitivity protein detection method based on nanoparticles and enzyme-linked immunosorbent assays.

Zhiwen, T et al., (2011), Enzyme-mimic activity of ferric nanon-core residing in ferritin and its biosensing applications. Analytical Chemistry, 83(22), pp. 8611-8616 relates to nanoscale globular protein cages encapsulating a ferric core.

Fan, J. et al., (2011), Direct evidence for catalase and peroxidase activities of ferritin-platinum nanoparticles. Biomaterials, 32(6), pp. 1611-1618 relates to use of apoferritin as a nucleation substrate.

### Description of the Invention

The present invention provides a bifunctional tumor diagnostic reagent, characterized in that it consists of a protein shell for specifically recognizing cancer tissues and/or cancer cells and an inorganic nano-core having the catalytic activity of a peroxidase, wherein the protein shell is selected from heat shock protein, DNA-binding protein, pteridine synthase, or a viral protein shell having a nano-cavity structure, wherein the inorganic nano-core is selected from a magnetic iron oxide nanoparticle, an iron sulfide nanoparticle, a noble metal-blended iron oxide nanoparticle, a double-metal alloy nanoparticle, or a cerium oxide nanoparticle.

Moreover, present invention also relates to a kit for tumor diagnosis, comprising a bifunctional tumor diagnostic reagent and a substrate of a peroxidase, wherein the bifunctional tumor diagnostic reagent consists of a protein shell for specifically recognizing cancer tissues and/or cancer cells and an inorganic nano-core having the catalytic activity of a peroxidase, wherein the inorganic nano-core is selected from a magnetic iron oxide nanoparticle, an iron sulfide nanoparticle, a noble metal-blended iron oxide nanoparticle, a double-metal alloy nanoparticle, or a cerium oxide nanoparticle.

Therefore, in an aspect, the present invention provides a bifunctional tumor diagnostic reagent, characterized in that it consists of a protein shell which specifically recognizes cancer tissues and/or cancer cells and an inorganic nano-core which has the catalytic activity of a peroxidase.

The protein shell specifically recognizing cancer tissues and/or cancer cells according to the present invention may be selected from various known proteins specifically recognizing cancer tissues and/or cancer cells in the art. In some embodiments, the protein shell of the present invention is a genetically recombinant or natural apoferritin. In some embodiments, the apoferritin may be self-assembled from 12 or 24 heavy chain subunits and light chain subunits at any ratio. In the present invention, the natural apoferritin may be derived from an eukaryotic or a prokaryotic organism, preferably from a mammal. In some embodiments, the protein shell of the present invention is a genetically recombinant ferritin consisting of only heavy chain subunits.

In some embodiments, the protein shell of the present invention is a viral protein shell comprising cowpea chlorotic mottle virus (CCMV, with a diameter of 28 nm) and cowpea mosaic virus protein (CMV, with a diameter of 31 nm).

In some embodiments, the protein shell of the present invention is chemically coupled or genetically fused to a target molecule and/or a signal molecule. In some of such embodiments, the target molecule according to the present invention comprises polypeptide or nucleic acid aptamer. In some of such embodiments, the target molecule according to the present invention is an antibody. In some embodiments, the signal molecule according to the present invention comprises fluorescence and radioactive nuclide.

In some embodiments, the inorganic nano-core of the present invention has the catalytic activity of a peroxidase, selected from magnetic iron oxide nanoparticles, iron sulfide nanoparticles, noble metal-blended iron oxide nanoparticles, double-metal alloy nanoparticles, or cerium oxide nanoparticles.

In some embodiments, the protein shell of the present invention is a genetically recombinant or natural apoferritin. In some embodiments, the apoferritin may be self-assembled from 12 or 24 heavy chain subunits and light chain subunits at any ratio.

### The Description of Drawings

Now the preferred embodiments of the present invention are described by referring to the following drawings:

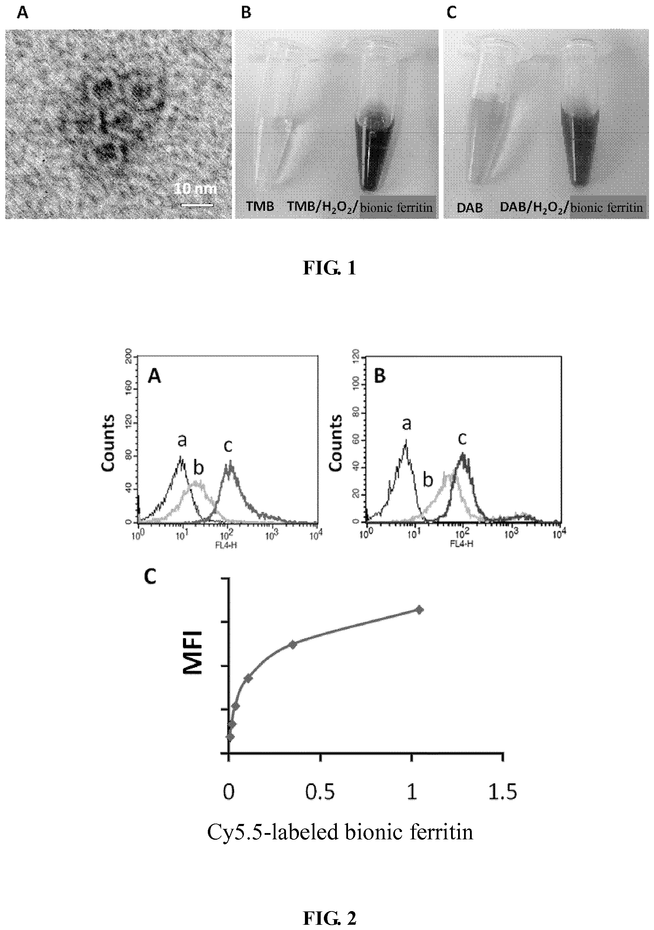

Fig. 1: A). the TEM photos of the bionic ferritin; B). the bionic ferritin has the catalytic activity of a peroxidase to catalyze HRP substrate, TMB, for color development; C). the bionic ferritin has the catalytic activity of a peroxidase to catalyze HRP substrate, DAB, for color development.
Fig. 2: the analysis of the interaction of colon rectal cancer cells HT29 with the bionic ferritin by flow cytometry. A). the competitive binding of the cell surface receptors; a: PBS control, b: staining with Cy5.5 labeled-bionic ferritin, c: the cell surface receptors are blocked with excessive un-labeled bionic ferritin; B). excessive anti-transferrin receptor 1 (TfR1) antibody competitively binds to the site of the bionic ferritin receptor, a: PBS control, b: Cy5.5 labeled-bionic ferritin, c: the sites of the receptor are blocked with excessive anti-TfR1 antibody; C). the cell-binding saturation curve of the bionic protein.

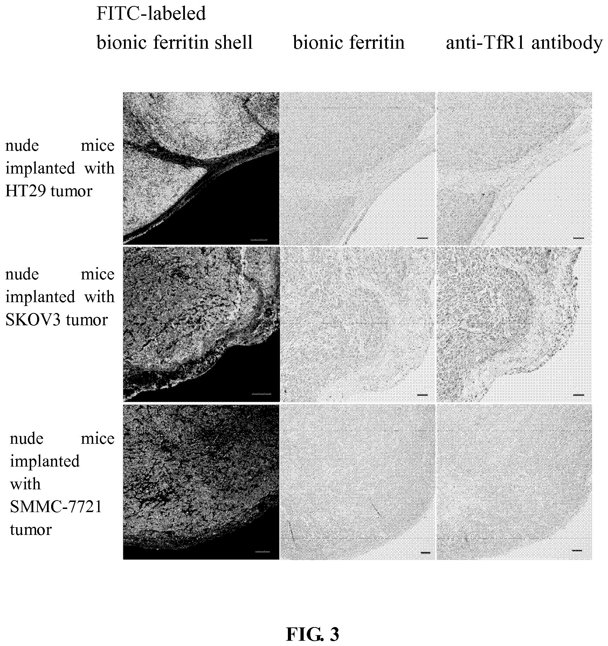

Fig. 3: the bionic ferritin specifically recognizes implanted tumor in mice. The staining region of the tumor tissue and the staining effect are comparable with those of an antibody (scale: 100 µm).

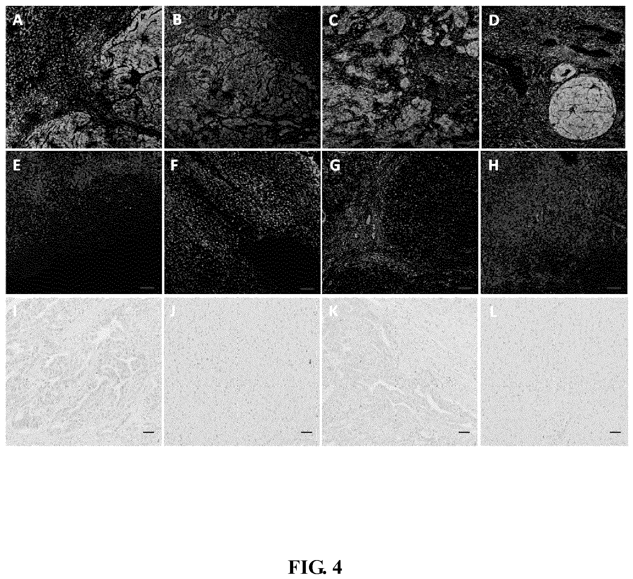

Fig. 4: the bionic ferritin specifically recognizes human hepatocellular carcinoma tissue sections, and does not recognize normal liver tissue, hepatitis or cirrhosis tissues. A)-D). four cases of hepatocellular carcinoma; E). normal liver tissue; F). the chronic inflammation of liver tissues; G). nodular cirrhosis; H). biliary cirrhosis; I). hepatocellular carcinoma; J). normal liver tissue; K). hepatocellular carcinoma; L). the chronic inflammation of liver tissues (scale: 100 µm).

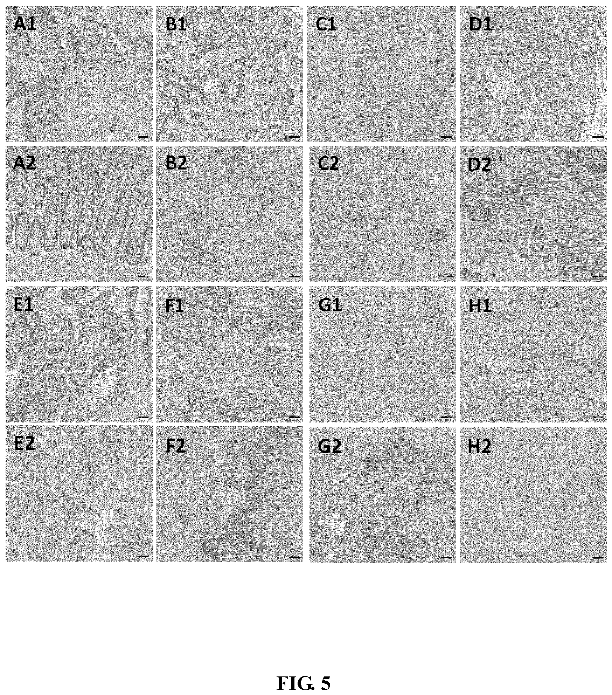

Fig. 5: the staining of various human cancerous tissues and normal tissues with the bionic ferritin. A1). colon cancer, stage II; A2). colon mucous membrane tissue; B1). mammary non-specific infiltrative duct carcinoma, stage I; B2). breast fibroadenosis; C1). ovary papillary serous adenocarcinoma, stage III; C2). ovary tissue; D1). prostate cancer, stage 4; D2). prostate smooth muscle tissue; E1). pulmonary squamous cell carcinoma, stage III; E2). pulmonary tissues companied with congestion; F1). esophagus cell cancer, stage III; F2). chronic esophagus inflammation companied with epithelial proliferation; G1). thymic carcinoma; G2). thymic tissue; H1). cervix squamous cell carcinoma, stage III; H2)., chronic cervix inflammation.

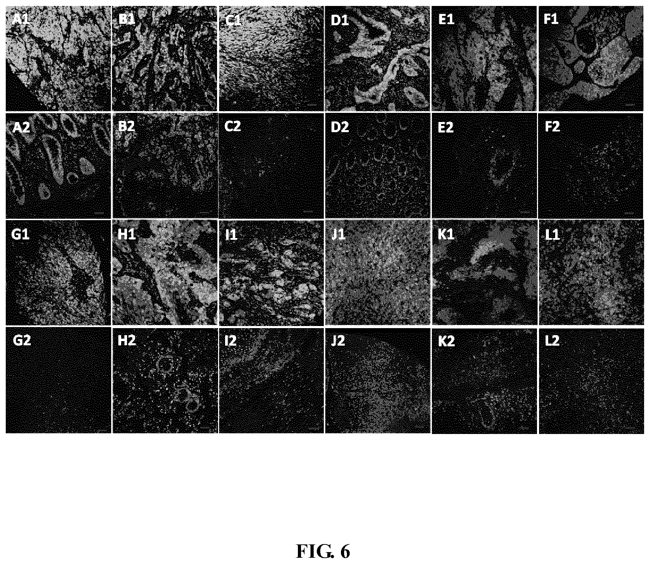

Fig. 6: Fluorescence-labeled bionic ferritin shell specifically recognizes various human cancerous tissues. A1). colon cancer, stage III; A2). chronic inflammation of colon mucous membrane companied with epithelial proliferation; B1). mammary non-specific infiltrative duct carcinoma, stage I; B2). breast tissue; C1). ovary papillary serous adenocarcinoma, stage III; C2). ovary tissue; D1). colon adenocarcinoma, stage II; D2). colon mucous membrane tissue; E1). prostate cancer, stage 4; E2). prostate proliferation companied with PIN, stage I; F1). pulmonary squamous cell carcinoma, stage III; F2). pulmonary tissues; G1). ovary papillary serous adenocarcinoma, stage III; G2). ovary tissue; H1). pulmonary squamous cell carcinoma, stage III; H2). pulmonary tissues companied with congestion; I1). esophagus squamous cell cancer, stage III; I2). chronic esophagus inflammation companied with epithelial proliferation; J1). thymic carcinoma; J2). thymic tissue; K1). cervix squamous cell carcinoma, stage II; K2). cervix tissue; L1). cervix squamous cell carcinoma, stage III; L2). chronic cervix inflammation (scale: 50 µm).

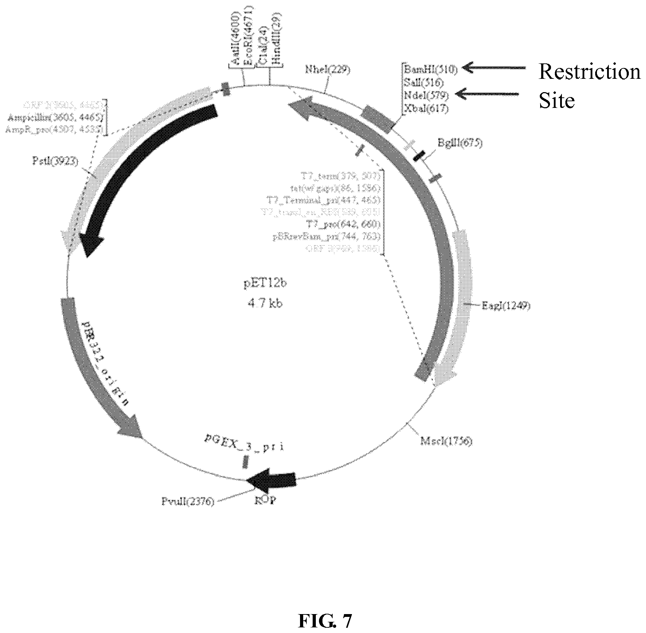

Fig. 7: the map of the plasmid used in the present invention.

### Examples

### Example 1. The preparation of the bionic ferritin.

The protein shell consisting of only the full heavy chain of human ferritin was obtained by a genetic recombination method. The iron ions were trans-loaded into the protein shell by the means of human biomineralization, and then the loaded iron ions were oxidized to iron oxide nanoparticles. We bionically synthesized a human ferritin which consists of only the full heavy chain of human ferritin and which encloses a magnetic iron oxide nano-core. The protein is named the bionic ferritin and it was used for tumor diagnosis.

The specific protocols were as follows:
At first, we constructed the recombinant plasmid pET12b-HFn containing the gene sequence which encodes only heavy chain subunit of human ferritin (Santambrogio P. et al.,(2000), Protein Expr. Purif., 19: 212-218). The whole cDNA library of human skeletal muscle was purchased from Invitrogen (D8090-01, Carlsbad, CA, USA). The gene encoding only the heavy chain of human ferritin was isolated and amplified from the cDNA library via PCR. The two primers were designed as follows: forward PCR primer: 5'-A GTC GCC CAT ATG ACG ACC GCG TCC-3' (Nde I site was underlined), with seven protection bases for enzyme digestion added, reverse PCR primer: 5' -GCC GGA TCC TTA GCT TTC ATT ATC AC -3' (BamHI site was underlined), with three protection bases for enzyme digestion added. 552 bp amplified gene product was the target gene product encoding only the heavy chain of human ferritin, and the sequence was shown below:

Subsequently, the amplified PCR product and pET-12b plasmid (Novagen, Inc., Madison, WI, USA) were digested with the restriction enzymes NdeI and BamHI. The digested PCR product was ligated to pET-12b vector to produce the recombinant plasmid pET12b-HFn containing the gene sequence of only the heavy chain subunit of human ferritin. The map of the plasmid was shown in the figure below, in which the enzyme sites were marked with red arrows, and the antibiotics resistance was Amp.

The resultant plasmid pET12b-HFn was used to transfect an expression strain, BL21DE3 (commercially available from Beijing Transgen Biotech. Co. Ltd, China) to express the heavy chain subunit of ferritin. The bacteria culture expressing the target protein was disrupted under ultrasound, and the debris of *E. coli* was removed by centrifugation. The supernatant was heated at 65 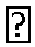 for 10 min. The impurity proteins were precipitated and removed by centrifugation. The resultant supernatant was separated and purified on the exclusion chromatography Sepharose 4B column to produce the recombinant heavy chain subunit of ferritin, which was tested for purity by electrophoresis and for protein concentration by BCA assay.

The biomineralization of the bionic ferritin was briefly described as follows:
100 mM of sodium chloride solution was subjected to degasification treatment, and was put into a sealed reactor full of nitrogen gas together with the purified ferritin protein shell. The reaction temperature was maintained at 65°C, pH was kept at 8.5. (NH₄)₂Fe(SO₄)₂·6H₂O was added at the ratio of 1000-5000 Fe ions/ferritin shell molecule. Hydrogen peroxide, as an oxidant, was added together with Fe ion at a ratio of H₂O₂:Fe²⁺=1:3. After the addition of hydrogen peroxide and Fe ion, the reaction was kept for 5 min. 200 µl of 300 mM sodium citrate was added to complex the remaining Fe ion. The principle for generating magnetic bionic ferritin was shown in Formula (1) listed below. The product was collected and purified via exclusion chromatography. The denatured impurity proteins were removed and the magnetized bionic ferritin consisting of only the heavy chain subunit was obtained.

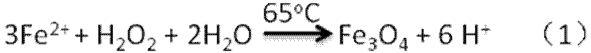

Fig. 1A was the TEM photos of the synthesized bionic ferritin. From this figure, it could be seen that the bionic ferritin was dispersed homogeneously; the outer diameter of the protein shell was about 12 nm, and the inner diameter of the protein shell was about 8 nm.

### Example 2. The peroxidase activity of the bionic ferritin.

According to the report of our research group, the magnetic iron oxide nanoparticles had a similar catalytic activity to a peroxidase (Gao L, et al. (2007) Nature Nanotech., 2: 577-583.). Because the bionic ferritin enclosed an iron oxide core, it is supposed to have a peroxidase-like activity. We tested the enzyme activity of the bionic ferritin with a substrate of HRP. The assay was specifically described as follows: adding 30% of H₂O₂ and TMB or DAB in the bionic ferritin to observe the change of color. As shown in Fig. 1B, after the bionic ferritin and H₂O₂ were added to TMB, it turned to dark blue, and after the bionic ferritin and H₂O₂ were added to DAB, it turned to dark brown. The color reaction was the same as that of HRP, indicating that the bionic ferritin had a similar peroxidase activity.

### Example 3. The bionic ferritin binds to tumor cells.

In order to investigate the binding of the bionic ferritin to human tumor cells, the common human tumor cells were selected to incubate with the bionic ferritin labeled with a fluorescent molecule, and the binding of the bionic ferritin to each kind of tumor cells was detected by flow cytometry.

The experimental method was as follows: according to the labeling method provided in the instructions, the bionic ferritin was labeled with NHS-activated Cy5.5 (Cy5.5-NHS, GE Healthcare); each strain of cells was cultured to the density of about 1×10⁵, and digested with trypsin; the cells were washed with 0.3% of BSA/PBS for three times; 50 µg/ml Cy5.5-labeled bionic ferritin was added and incubated at 4 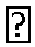 for 45 min; and then, the cells were washed with 0.3% of BSA/PBS for three times, and finally resuspended in PBS; the cell sample was tested for fluorescence via flow cytometry.

The result was shown in Table 1. The bionic ferritin had reaction activity with 11 cell strains of 12 cell strains to be tested, which were the cells of hepatocellular carcinoma, colorectal cancer, breast cancer, melanoma, monocyte lymphoma, cervix cancer, leukemia and prostate cancer, respectively, indicating that the bionic ferritin was capable of specifically binding to the majority of cancer cells.

**Table 1. The analysis of the reaction activity of the bionic ferritin with human tumor cells via flow cytometry.**

| **Cell line/catalog No.** | **Tumor type** | **reactivity** |
|---|---|---|
| SMMC-7721/(TCHu 52) | hepatocellular carcinoma | + |
| HT29/(HTB-38) | colorectal cancer | + |
| MDA-MB-231/(HTB-26) | breast cancer | + |
| MX-1/ (KG038) | breast cancer | - |
| A375/(CRL-1619) | melanoma | + |
| K562/(CCL-243) | human chronic myelogenous leukemia | + |
| HeLa/(CCL-2) | cervix cancer | + |
| SKOV3/(HTB-77) | ovary cancer | + |
| PC-3/(CRL-1435) | prostate cancer | + |
| U251/(TCHu 58) | glioma | + |
| U937/(CRL-1593.2) | lymphoma | + |
| Jurkatr/(TIB-152) | leukemia T cells | + |

| | | |
|---|---|---|
| * SMMC-7721 and U251 were commercially available from Chinese Academy of Science Type Culture Collection Cell Bank, MX-1 was commercially available from KeyGen Biotech. Co. Ltd, and the others were commercially available from ATCC. | | |

### Example 4. The interaction of the cancer cells with the bionic ferritin.

In order to investigate the binding of the bionic ferritin to the tumor cell surface receptors, the inventor incubated different concentrations of Cy5.5-labeled bionic ferritin with colorectal cancer cell HT29 to obtain the binding saturation curve of bionic ferritin. The receptor sites were blocked with excessive anti-TfR1 antibody to demonstrate that TfR1 mediated the interaction of bionic ferritin with cancer cells.

The experimental method was as follows: the cancer cells HT29 were cultured to the density of about 1×10⁵, and digested with trypsin; the cells were washed with 0.3% of BSA/PBS for three times; different concentrations of Cy5.5-labeled bionic ferritin were added and incubated at 4°C for 45 min; and then, the cells were washed with 0.3% of BSA/PBS for three times, and finally resuspended in PBS; the cell sample was tested for fluorescence via flow cytometry. A saturation curve was plotted based on the fluorescence intensity and the concentration of the bionic ferritin. The result was shown in Fig. 2C. The binding of the bionic ferritin to tumor cells is saturable, indicating that such binding was specific and had a high affinity.

Subsequently, based on the saturation curve, a suitable concentration of Cy5.5-labeled bionic ferritin was selected for the competition binding inhibition assay. Likewise, after the cells were cultured to the density of about 1×10⁵, digested with trypsin, and washed with buffers, a certain concentration of Cy5.5-labeled bionic ferritin and excessive unlabeled bionic ferritin were added to the cells at the same time, and incubated at 4°C for 45 min. The cells were washed for three times, and finally resuspended in PBS. The cell sample was tested for fluorescence via flow cytometry. The result was shown in Fig. 2A. The labeled bionic ferritin bound to the cells, but majority of them could be competed with excessive unlabeled bionic ferritin, further indicating that the binding of bionic ferritin to cancer cells was specific, with only a small number of non-specific binding molecules left (not being competed) on the cell surface.

Li L. et al., (2010) Proc. Natl. Acad. Sci. 107: 3505-3510, reported the co-receptor of human ferritin and transferrin, TfR1. In order to demonstrate that TfR1 mediates the interaction of bionic ferritin with cancer cells, we used excessive anti-TfR1 antibody to compete with Cy5.5-labeled bionic ferritin for the cell surface receptor. The specific experimental method was as follows: HT29 cells were cultured to the density of about 1×10⁵, and digested with trypsin; the cells were washed for three times; excessive mice anti-human TfR1 antibody CD71 (BD Pharmingen) and Cy5.5-labeled bionic ferritin were added at the same time and incubated at 4°C for 45 min; the cells were washed with 0.3% of BSA/PBS for three times, and finally resuspended in PBS; the cell sample was tested for fluorescence via flow cytometry.

The result was shown in Fig. 2B. About 50% of the bionic ferritin was competed, indicating that TfR1 mediated the interaction of the bionic ferritin with cancer cells.

### Example 5. Specific detection of an implanted tumor in mice using the bionic ferritin.

In order to investigate the recognition and staining capacity of the bionic ferritin to tumor tissues, we incubated it with the nude mice-implanted tumor tissue, and compared with the result of conventional immunohistochemisty method using antibodies.

The specific experimental method was as follows:
The paraffin section of mice-implanted tumor was made by the following method: the cultured HT-29, SMMC-7721 and SKOV-3 tumor cells (ATCC) were collected, washed with serum free medium, and resuspended in PBS buffer. The female BALB/c mice (Beijing Experimental Animal Center, China) were subcutaneously injected with 5×10⁶ HT-29, SMMC-7721 or SKOV-3 cells at shoulder site. The mice were sacrificed when the tumor grew to 0.4 to 0.6 cm in diameter, and the tumor was taken and fixed with 4% of paraformaldehyde for 12 to 24 hours, washed with 70% of ethanol, and gradually dehydrated with 70-80-90-95-95-100% of ethanol for 35 min, immersed into 1:1 ethanol/xylene solution for 5 min, taken from ethanol/xylene solution and permeabilized twice by immersing into xylene for 20 min, put into hot-molten paraffin (melting point of 56-58°C) for 4 hours. The wax-dipped tissue was taken and transferred to a paper box containing molten paraffin, with the incisal surface downwards. It was embedded after the surface of paraffin was coagulated, and solidified by immersing into cold water to produce a paraffin block. The paraffin-embedded tissue was cut into sections with 5 µm thickness by Leica paraffin slicer. The section was spread on a slide at 42°C, dried at room temperature and baked at 50 °C for 6 hours to produce the paraffin sections of mice-implanted HT-29, SMMC-7721 and SKOV-3 tumors, respectively.

The method for staining tumor-implanted tissues with anti-TfR1 antibody CD71 was briefly described as follows: the paraffin sections of the implanted tumor were deparaffinaged to water, washed with PBS for three times, incubated for 30 min by adding methanol solution containing 0.3% of H₂O₂ to inactivate the endogenous peroxidase in the tissue; 1 mM citric acid antigen restoration solution was added, and heated in microwave oven for 30 min for antigen restoration; after rinsed with deionized water, it was blocked with 5% of ordinary sheep serum at 37°C for 1 hour; Anti-TfR1 antibody CD71 (BD Pharmingen) was added and incubated at 4°C over night; after rinsed with PBS, 1000-fold diluted secondary antibody was added and incubated at 37 °C for 1 hour, and then rinsed with PBS; 2000-fold diluted HRP-labeled tertiary antibody was added and incubated at 37 °C for 1 hour, and then rinsed with PBS; one droplet of each of Buffer, H₂O₂ and DAB contained in DAB kit was added into 1 ml deionized water, mixed uniformly and dropped onto the tissue specimen on the slide to develop for 5 min; it was rinsed with deionized water, counter-stained with haematoxylin, dehydrated and mounted, and then observed with microscope for imagining.

The method for staining mice-implanted tumor tissue with bionic ferritin was briefly described as follows: after deparaffinage treatment of the paraffin sections of the implanted tumor, inactivating of the endogenous peroxidase in the tissue, restoring of the antigen, as well as blocking with the serum, the bionic ferritin was added, and incubated at room temperature for 45 min, the specimen was rinsed with PBS, then stained with DAB, counter-stained with haematoxylin, conventionally dehydrated and mounted, and then observed under microscope.

**Staining mice-implanted tumor with FITC labeled bionic ferritin:** According to the method recorded in the instructions, the bionic ferritin was labeled with fluorescein isothiocyanate FITC (Sigma-Aldrich). After the paraffin section of the implanted tumor tissue was blocked with serum, the FITC labeled bionic ferritin was added and incubated at room temperature for 45 min, rinsed twice with PBS, and the nuclei were stained with DAPI for 10 min. The quencher was added and the section was mounted and observed under fluorescent confocal microscopy.

The result was shown in Fig. 3. The bionic ferritin was capable of specifically recognizing the tumor cells in the tissues. The stained region was consistent with that of antibody CD71. The staining effect of the tumors was comparable to that of the antibody. The change of cell morphology could be clearly distinguished. The fluorescent confocal result which showing the recognition of tumor tissues by the bionic ferritin further demonstrated that the bionic ferritin shell can achieve the specific recognition of a tumor. As compared with the conventional immunohistochemisty method, the bionic ferritin can achieve the specific recognition for the antigen and the staining in one step, without the need of multiple steps of incubation with a primary antibody, a secondary antibody and a tertiary antibody, and of modification with a signal molecule such as HRP enzyme and the like. And thus, it is simple, convenient and quick, as compared with the conventional immunohistochemisty method.

### Example 6. The bionic ferritin recognizes and stains the clinic human liver cancer tissues.

Liver tissue specimens were obtained from Beijing Anzhen Hospital and Beijing Tumor Hospital. All the liver tissue specimens were the bulk specimens of the surgically ablated tumor or adjacent tissues. All the tissues were embedded in paraffin.

The method for staining the tissue sections with bionic ferritin was briefly described as follows: after deparaffinage treatment of the paraffin-embedded liver cancer tissue and normal liver tissue, inactivating of the endogenous peroxidase in the tissue, restoring of the antigen, as well as blocking with the serum, the bionic ferritin was added, and incubated at room temperature for 45 min, the specimen was rinsed with PBS, then stained with DAB, counter-stained with haematoxylin, conventionally dehydrated and mounted, and then observed under microscope.

The method for fluorescent staining tissue sections with bionic ferritin was briefly described as follows: after deparaffinage treatment of the paraffin-embedded liver cancer tissue and normal liver tissue as well as blocking with serum, the FITC-labeled bionic ferritin shell was added and incubated at room temperature for 45 min, the specimen was rinsed twice with PBS, and the nuclei were stained with DAPI for 10 min; the quencher was added and the section was mounted and observed under fluorescent confocal microscopy.

The result was shown in Fig. 4. The bionic ferritin was capable of specifically recognizing the cancerous cells in liver tissue, and it did not stain the normal tissue adjacent to the tumor, and did not recognize the tissues such as chronic inflammation of liver tissue, nodular cirrhosis, biliary cirrhosis and the like. In all the tested hepatocellular carcinoma tissues, the positive ratio was 54/55, while the positive ratio in the normal liver tissue, hepatitis and cirrhosis was 7/45, indicating that the bionic ferritin was capable of specifically recognizing liver cancer tissues.

### Example 7. The bionic ferritin specifically recognizes various human cancer tissues.

In order to systemically investigate the capacity of bionic ferritin to specifically recognize the cancerous tissues and human normal tissues, we selected paraffin section specimens of human tumor tissues and 48 cases of human normal tissues or inflammation tissues for bionic ferritin staining. The patient tissue specimens were obtained from Beijing Anzhen Hospital, Beijing Tumor Hospital and Xi'an Aomei Biotech. Co. Ltd. All the liver tissue specimens were the bulk specimens of the surgically ablated tumor or adjacent tissues. All the tissues were embedded in paraffin.

The method for staining the tissue sections with bionic ferritin was briefly described as follows: after deparaffinage treatment of the paraffin-embedded human tumor tissue, normal tissue or related inflammation tissue, inactivating of the endogenous peroxidase in the tissue restoring of the antigen, as well as blocking with serum, the bionic ferritin was added, and incubated at room temperature for 45 min, the specimen was rinsed with PBS, stained with DAB, counter-stained with haematoxylin, conventionally dehydrated and mounted, and then observed under microscope. The tissue-recognition results were shown in Fig. 5 and Table 2.

The method for fluorescent staining tissue sections with bionic ferritin was briefly described as follows: after deparaffinage treatment of the paraffin-embedded tumor tissue, normal tissue or inflammation tissue as well as blocking with serum, the FITC-labeled bionic ferritin shell was added and incubated at room temperature for 45 min, the specimen was rinsed twice with PBS, and the nuclei were stained with DAPI for 10 min; the quencher was added and the section was mounted and observed under fluorescent confocal microscopy. The result was shown in Fig. 6.

As shown in Figs. 5 and 6, the bionic ferritin exhibited strong binding to colon cancer, breast cancer, ovary cancer, colon adenocarcinoma, pulmonary squamous cell carcinoma, prostate cancer, esophagus squamous cell carcinoma, thymic carcinoma and cervix squamous cell carcinoma, and like, while it exhibited no relative binding to the corresponding chronic colon inflammation, breast tissue, ovary tissue, colon mucous membrane tissue, lung tissue, prostate proliferation, chronic esophagus inflammation, thymic tissue, cervix tissue and chronic cervix inflammation and the like. The analysis result showed in Table 2 indicated that the bionic ferritin exhibited excellent staining sensitivity and specificity to hepatocellular carcinoma (54/55), pulmonary squamous cell carcinoma (50/52), thymic carcinoma (4/4), cervix squamous cell carcinoma (4/4), esophagus cancer (3/3), colon cancer (6/6) and ovary cancer (4/4) and the like. The tissue-recognition effect shown in Figs. 5 and 6 demonstrated that the bionic ferritin is capable of specifically recognizing various human cancerous tissues, especially strongly staining and recognizing hepatocellular carcinoma, lung cancer, thymic carcinoma, cervix cancer, ovary cancer and colon cancer, while it exhibited no recognition to the corresponding normal or inflammation tissues. As compared with the conventional immunohistochemisty method, the method for identifying cancerous tissues based on bionic ferritin is capable of specifically recognizing tumors and staining in one step, without the need of multiple steps of incubation with a primary antibody, a secondary antibody and a tertiary antibody, and of labeling with an enzyme such as HRP etc. or a signal molecule, the manipulation thereof is simple and moreover, the staining effect thereof to the tumor tissues is comparable to that of the immunohistochemisty method.

**Table 2. The analysis of the specific recognition of bionic ferritin to clinical tumor tissues and the corresponding normal tissues.**

| **Tumor tissues** | **Positive cases/total cases** | **Normal tissues/inflammation tissues** | **Positive cases/total cases** |
|---|---|---|---|
| hepatocellular carcinoma | 54/55 | normal liver/ hepatitis /cirrhosis | 7/45 |
| pulmonary squamous cell carcinoma | 50/52 | pulmonary tissue | 1/56 |
| thymic carcinoma | 4/4 | thymic tissue | 0/4 |
| cervix squamous cell carcinoma | 28/28 | cervix tissue /chronic inflammation | 0/52 |
| prostate cancer | 22/22 | prostate smooth muscle/prostate proliferation | 2/16 |
| mammary non-specific infiltrative duct carcinoma | 4/5 | breast tissue/breast fibrosis /proliferative fibrous tissue/ breast adipose tissue | 1/4 |
| ovary cancer | 55/56 | ovary tissue /ovary epithelial proliferation | 1/16 |
| esophagus squamous cell cancer | 3/3 | esophagus smooth muscle/chronic inflammation companied with epithelial proliferation | 0/5 |
| colon adenocarcinoma | 23/23 | colon mucous membrane tissue / chronic inflammation of colon mucous membrane | 1/41 |

### References:

1. Shi SR., (2008), Evaluation of the value of frozen tissue section used as "Gold Standard" for immunohistochemistry, Am. J. Clin. Pathol., 129:358-366.
2. Taylor CR., (2006), Standardization in immunohistochemistry: the role of antigen retrieval in molecular morphology, Biotech Histochem., 81:3-12.
3. Larsson L., (1988), Immunocytochemistry: Theory and Practice. Boca Raton, FL: CRC Press, 41-73.
4. Turyanska L., et al. (2009), The Biocompatibility of Apoferritin-Encapsulated PbS Quantum Dots, Small, 5:1738-1741.
5. Meldrum FC., et al. (2009), Magnetoferritin:in vitro synthesis of a novel magnetic protein, Science, 257:522-523.
6. Galvez N., et al. (2006), Apoferritin-encapsulated Ni and Co superparamagnetic nanoparticles, J. Mater. Chem., 16:2757-2761.
7. Hennequin B. et al. (2008), Aqueous near-infrared fluorescent composites based on apoferritin-encapsulated PbS quantum dots, Adv .Mater.,20:3592-3593.
8. Yamashita I., et al. (2004), Bio-template synthesis of uniform CdSe nanoparticles using cage-shaped protein,apoferritin, Chem. Lett., 33:1158-1159.
9. Uchida M., et al. (2006), Targeting of Cancer Cells with Ferrimagnetic ferritin cage nanoparticles, J. Am. Chem. Soc., 128: 16626-16633.
10. Lin X., et al. (2011), Chimeric ferritin nanocages for multiple function loading and multimodal imaging, Nano Lett., 11: 814-819,
11. Geninatti CS., et al. (2006) Magnetic resonance visualization of tumor angiogenesis by targeting neural cell adhesion molecules with the highly sensitive gadolinium-loaded apoferritin probe, Cancer Res., 66: 9196-201.
12. Gao L., et al. (2007), Intrinsic peroxidase-like activity of ferromagnetic nanoparticles, Nature Nanotech., 2:577-583.
13. Chinese Patent No. 200610057413.9
14. Theil EC., (1987), Ferritin: structure, gene regulation, and cellular function in animals, plants, and microorganisms. Annu. Rev. Biochem., 56:289-315.
15. Harrison PM., et al. (1996) The ferritins: molecular properties, iron storage function and cellular regulation, Biochim. Biophys. Acta, 1275:161-203.
16. Levi S., et al. (1988), Mechanism of ferritin iron uptake-Activity of the H-chain and deletion mapping of the ferro-oxidase site-A study of iron uptake and ferro-oxidase activity of human-liver, recombinant H-chain ferritins, and of 2H-chain deletion mutants, J. Biol. Chem., 263:18086-18092.
17. Ford GC., et al. (1984), Ferritin: Design and formation of an iron-storage molecule, Philos Trans. R. Soc. Lond B Biol. Sci., 304:551-565.
18. Fargion S, et al. (1988) Characteristics and expression of binding sites specific for ferritin H-chain on human cell lines. Blood 71:753-757.
19. Moss D, et al.(1992) Characterization of the ferritin receptors of human T lymphoid (MOLT-4) cells, J. Lab Clin. Med. 119:273-279.
20. Bretscher MS, et al. (1983) Distribution of ferritin receptors and coated pits on giant HeLa cells, EMBO J. 2:599-603.
21. Takami M, et al. (1986) Human placental ferritin receptor. Biochim. Biophys Acta 884:31-38.
22. Fargion S, et al. (1991) Specific binding sites for H-ferritin on human lymphocytes: Modulation during cellular proliferation and potential implication in cell growth control, Blood 78:1056-1061.
23. Moss D., et al. (1992) Functional roles of the ferritin receptors of human liver, hepatoma, lymphoid and erythroid cells, J. Inorg. Biochem. 47:219-227.
24. Li L., et al. (2010), Binding and uptake of H-ferritin are mediated by human transferrin receptor-1, Proc. Natl. Acad. Sci. U S A, 107: 3505-3510.
25. Larrick JW., et al. (1979), Modulation of cell surface iron transferrin receptors by cellular density and state of activation, J. Supramol. Struct., 11:579-586.
26. Daniels TR., et al. (2006), The transferrin receptor part I: biology and targeting with cytotoxic antibodies for the treatment of cancer, Clin. Immunol., 121: 144-158.
27. Ryschich E., et al. (2004), Transferrin receptor is a marker of malignant phenotype in human pancreatic cancer and in neuroendocrine carcinoma of the pancreas, Eur. J. Caner, 40:1418-1422.
28. Santambrogio P., et al. (2000), Functional and immunological analysis of recombinant mouse H- and L-ferritins from Escherichia coli, Protein Expr. Purif., 19: 212-218.

## Claims

1. A bifunctional tumor diagnostic reagent, **characterized in that** it consists of a protein shell for specifically recognizing cancer tissues and/or cancer cells and an inorganic nano-core having the catalytic activity of a peroxidase, wherein the protein shell is selected from heat shock protein, DNA-binding protein, pteridine synthase, or a viral protein shell having a nano-cavity structure, wherein the inorganic nano-core is selected from a magnetic iron oxide nanoparticle, an iron sulfide nanoparticle, a noble metal-blended iron oxide nanoparticle, a double-metal alloy nanoparticle, or a cerium oxide nanoparticle.

2. The bifunctional tumor diagnostic reagent according to claim 1, wherein the viral protein shell having a nano-cavity structure comprises cowpea chlorotic mottle virus and cowpea mosaic virus protein.

3. The bifunctional tumor diagnostic reagent according to any one of claims 1 to 2, **characterized in that** the protein shell comprises a protein shell chemically coupled or genetically fused to a targeting molecule and/or a signal molecule, wherein the targeting molecule comprises a polypeptide or a nucleic acid aptamer, and wherein the polypeptide comprises an antibody, and wherein the signal molecule comprises a fluorescent molecule, a radioactive nuclide and an enzyme.

4. A kit for tumor diagnosis, comprising a bifunctional tumor diagnostic reagent and a substrate of a peroxidase, wherein the bifunctional tumor diagnostic reagent consists of a protein shell for specifically recognizing cancer tissues and/or cancer cells and an inorganic nano-core having the catalytic activity of a peroxidase, wherein the inorganic nano-core is selected from a magnetic iron oxide nanoparticle, an iron sulfide nanoparticle, a noble metal-blended iron oxide nanoparticle, a double-metal alloy nanoparticle, or a cerium oxide nanoparticle.

5. The kit according to claim 4, **characterized in that** the protein shell comprises apoferritin, heat shock protein, DNA-binding protein, pteridine synthase, and a viral protein shell having a nano-cavity structure, wherein the viral protein shell having a nano-cavity structure comprises cowpea chlorotic mottle virus and cowpea mosaic virus protein.

6. The kit according to claim 5, **characterized in that** the apoferritin is self-assembled from 12 or 24 heavy chain subunits and light chain subunits at any ratio.

7. The kit according to claim 5, **characterized in that** the apoferritin is a natural apoferritin or genetically recombinant apoferritin, wherein the natural apoferritin is derived from an eukaryotic or a prokaryotic organism.

8. The kit according to claim 7, wherein the natural apoferritin is derived from a mammal.

9. The kit according to claim 4, **characterized in that** the protein shell comprises a protein shell chemically coupled or genetically fused to a targeting molecule and/or a signal molecule, wherein the targeting molecule comprises a polypeptide or a nucleic acid aptamer, and wherein the polypeptide comprises an antibody, and wherein the signal molecule comprises a fluorescent molecule, a radioactive nuclide and an enzyme.

10. The kit according to claim 4, **characterized in that** the protein shell is a generically recombinant ferritin, consisting of heavy chain subunits, and the inorganic core is a magnetic iron oxide nanoparticle.

## Patentansprüche

1. Bifunktionelles Tumordiagnose-Reagenz, **dadurch gekennzeichnet, dass** es aus einer Proteinhülle zum spezifischen Erkennen von Krebsgeweben und/oder Krebszellen und einem anorganischen Nanokern mit der katalytischen Aktivität einer Peroxidase besteht, wobei die Proteinhülle aus Hitzeschockprotein, DNA-bindendem Protein, Pteridinsynthase, oder einer Virusproteinhülle mit einer Nano-Hohlraumstruktur ausgewählt ist, wobei der anorganische Nanokern aus einem magnetischen Eisenoxid-Nanoteilchen, einem Eisensulfid-Nanoteilchen, einem mit Edelmetall gemischten Eisenoxid-Nanoteilchen, einem Doppelmetalllegierung-Nanoteilchen oder einem Ceriumoxid-Nanoteilchen ausgewählt ist.

2. Bifunktionelles Tumordiagnose-Reagenz gemäß Anspruch 1, wobei die Virusproteinhülle mit einer Nano-Hohlraumstruktur Augenbohne-ChloroseMottle-Virus- und Augenbohne-Mosaik-Virusprotein umfasst.

3. Bifunktionelles Tumordiagnose-Reagenz gemäß einem der Ansprüche 1 bis 2, **dadurch gekennzeichnet, dass** die Proteinhülle eine Proteinhülle umfasst, die mit einem Zielmolekül und/oder einem Signalmolekül chemisch gekoppelt oder genetisch fusioniert ist, wobei das Zielmolekül ein Polypeptid oder ein Nucleinsäureaptamer umfasst, und wobei das Polypeptid einen Antikörper umfasst, und wobei das Signalmolekül ein fluoreszierendes Molekül, ein radioaktives Nuklid und ein Enzym umfasst.

4. Kit zur Tumordiagnose, umfassend ein bifunktionelles Tumordiagnose-Reagenz und ein Substrat einer Peroxidase, wobei das bifunktionelle Tumordiagnose-Reagenz aus einer Proteinhülle zum spezifischen Erkennen von Krebsgeweben und/oder Krebszellen und einem anorganischen Nanokern mit der katalytischen Aktivität einer Peroxidase besteht, wobei der anorganische Nanokern aus einem magnetischen Eisenoxid-Nanoteilchen, einem Eisensulfid-Nanoteilchen, einem mit Edelmetall gemischten Eisenoxid-Nanoteilchen, einem Doppelmetalllegierung-Nanoteilchen oder einem Ceriumoxid-Nanoteilchen ausgewählt ist.

5. Kit gemäß Anspruch 4, **dadurch gekennzeichnet, dass** die Proteinhülle Apoferritin, Hitzeschockprotein, DNA-bindendes Protein, Pteridinsynthase und eine Virusproteinhülle mit einer Nano-Hohlraumstruktur umfasst, wobei die Virusproteinhülle mit einer Nano-Hohlraumstruktur Augenbohne-Chlorose-Mottle-Virus- und Augenbohne-Mosaik-Virusprotein umfasst.

6. Kit gemäß Anspruch 5, **dadurch gekennzeichnet, dass** das Apoferritin aus 12 oder 24 schweren Kettenuntereinheiten und leichten Kettenuntereinheiten in einem beliebigen Verhältnis selbst zusammengesetzt ist.

7. Kit gemäß Anspruch 5, **dadurch gekennzeichnet, dass** das Apoferritin ein natürliches Apoferritin oder genetisch rekombinantes Apoferritin ist, wobei das natürliche Apoferritin von einem eukaryotischen oder einem prokaryotischen Organismus stammt.

8. Kit gemäß Anspruch 7, wobei das natürliche Apoferritin von einem Säugetier stammt.

9. Kit gemäß Anspruch 4, **dadurch gekennzeichnet, dass** die Proteinhülle eine Proteinhülle umfasst, die mit einem Zielmolekül und/oder einem Signalmolekül chemisch gekoppelt oder genetisch fusioniert ist, wobei das Zielmolekül ein Polypeptid oder ein Nucleinsäureaptamer umfasst, und wobei das Polypeptid einen Antikörper umfasst, und wobei das Signalmolekül ein fluoreszierendes Molekül, ein radioaktives Nuklid und ein Enzym umfasst.

10. Kit gemäß Anspruch 4, **dadurch gekennzeichnet, dass** die Proteinhülle ein genetisch rekombinantes Ferritin ist, das aus schweren Kettenuntereinheiten besteht, und der anorganische Kern ein magnetisches Eisenoxid-Nanoteilchen ist.

## Revendications

1. Réactif bifonctionnel de diagnostic de tumeur, **caractérisé en ce qu'**il est constitué d'une coque protéinique destinée à reconnaître spécifiquement des tissus cancéreux et/ou des cellules cancéreuses et d'un nano-noyau inorganique possédant l'activité catalytique d'une peroxydase, dans lequel la coque protéinique est choisie parmi une protéine de choc thermique, une protéine de liaison d'ADN, la ptéridine synthase ou une coque de protéine virale possédant une structure à nano-cavités, dans lequel le nano-noyau inorganique est choisi parmi une nanoparticule d'oxyde de fer magnétique, une nanoparticule de sulfure de fer, une nanoparticule d'oxyde de fer mélangé à un métal noble, une nanoparticule d'alliage à double métal ou une nanoparticule d'oxyde de cérium.

2. Réactif bifonctionnel de diagnostic de tumeur selon la revendication 1, dans lequel la coque de protéine virale possédant une structure à nanocavités comprend une protéine de virus marbrure chlorotique dolique et de virus de la mosaïque dolique.

3. Réactif bifonctionnel de diagnostic de tumeur selon l'une quelconque des revendications 1 à 2, **caractérisé en ce que** la coque protéinique comprend une coque protéinique couplée chimiquement ou fusionnée génétiquement à une molécule de ciblage et/ou à une molécule signal, dans lequel la molécule de ciblage comprend un polypeptide ou un aptamère d'acide nucléique et dans lequel le polypeptide comprend un anticorps et dans lequel la molécule signal comprend une molécule fluorescente, un radionucléide et une enzyme.

4. Trousse destinée au diagnostic de tumeur, comprenant un réactif bifonctionnel de diagnostic de tumeur et un substrat d'une peroxydase, dans laquelle le réactif bifonctionnel de diagnostic de tumeur est constitué d'une coque protéinique destinée à reconnaître spécifiquement des tissus cancéreux et/ou des cellules cancéreuses et d'un nano-noyau inorganique possédant l'activité catalytique d'une peroxydase, dans laquelle le nano-noyau inorganique est choisi parmi une nanoparticule d'oxyde de fer magnétique, une nanoparticule de sulfure de fer, une nanoparticule d'oxyde de fer mélangé à un métal noble, une nanoparticule d'alliage à double métal ou une nanoparticule d'oxyde de cérium.

5. Trousse selon la revendication 4, **caractérisée en ce que** la coque protéinique comprend de l'apoferritine, une protéine de choc thermique, une protéine de liaison d'ADN, de la ptéridine synthase et une coque de protéine virale possédant une structure à nano-cavités, dans laquelle la coque de protéine virale possédant une structure à nano-cavités comprend une protéine de virus marbrure chlorotique dolique et de virus de la mosaïque dolique.

6. Trousse selon la revendication 5, **caractérisée en ce que** l'apoferritine est auto-assemblée à partir de 12 ou 24 sous-unités de chaîne lourde et sous-unités de chaîne légère à n'importe quel rapport.

7. Trousse selon la revendication 5, **caractérisée en ce que** l'apoferritine est une apoferritine naturelle ou une apoferritine génétiquement recombinée, dans laquelle l'apoferritine naturelle est dérivée d'un organisme eucaryote ou procaryote.

8. Trousse selon la revendication 7, dans laquelle l'apoferritine naturelle est dérivée d'un mammifère.

9. Trousse selon la revendication 4, **caractérisée en ce que** la coque protéinique comprend une coque protéinique couplée chimiquement ou fusionnée génétiquement à une molécule de ciblage et/ou à une molécule signal, dans laquelle la molécule de ciblage comprend un polypeptide ou un aptamère d'acide nucléique et dans laquelle le polypeptide comprend un anticorps et dans laquelle la molécule signal comprend une molécule fluorescente, un radionucléide et une enzyme.

10. Trousse selon la revendication 4, **caractérisée en ce que** la coque protéinique est une ferritine génériquement recombinée, constituée de sous-unités de chaîne lourde et le noyau inorganique est une nanoparticule d'oxyde de fer magnétique.
